# EUROPÄISCHE PATENTSCHRIFT

(11) **EP 0 402 496 B1**
(45) Veröffentlichungstag und Bekanntmachung des Hinweises auf die Patenterteilung: **11.08.1993**
(21) Anmeldenummer: 89110700.5
(22) Anmeldetag: 13.06.1989
(51) Int. Cl.: C12M 1/00

(54) **Einrichtung zum Kultivieren von autotrophen Mikroorganismen**
Plant for cultivating autotrophic microorganisms
Installation pour la culture de microorganismes autotrophes

(43) Veröffentlichungstag der Anmeldung: 19.12.1990
(73) Patentinhaber: Institut für Getreideverarbeitung GmbH, 14558 Bergholz-Rehbrücke (DE)
(72) Erfinder: Horst, Franke, Dipl.-Chem., DDR-1580 Potsdam (DD); Köhler, Eva-Maria, Dipl.-Ing., DDR-1560 Potsdam (DD); Werner, Gabriela, Dipl.-Chem., DDR-1580 Potsdam (DD); Träger, Gisela, DDR-1560 Potsdam (DD); Hofmann, Tobias, Dipl.-Biochem., DDR-1590 Potsdam (DD); Storandt, Regina, Dipl.-Agr., DDR-1580 Potsdam (DD); Schmidt, Hans-Günther, Dipl.-Ing., DDR-6325 Ilmenau (DD); Fiedler, Joachim, Dipl.-Ing., DDR-6300 Ilmenau (DD); Weiz, Thomas, DDR-6312 Langewiesen (DD)
(74) Vertreter: Radwer, Dieter

(56) Entgegenhaltungen:
- GB-A- 2 205 581

## Beschreibung

Die Erfindung betrifft eine Einrichtung zum Kultivieren von autotrophen Mikroorganismen, die in der Lebensmittel- und Futtermittelindustrie oder auch in der Pharmazie Anwendung finden können.

Es ist bekannt, autotrophe Mikroorganismen zu kultivieren entweder in offenen Anlagen, die mittels Sonneneinstrahlung belichtet werden, oder es wird zur Herbeiführung der Photosynthese Kunstlicht angewendet. Es ist auch üblich, beide Belichtungsarten, z.B. im Tag-Nacht-Betrieb zu verwenden. Dabei geht es vor allem darum, die aufgewandte Lichtenergie in einem möglichst hohen Maße auszunutzen.
So ist es nach der DD-PS 219 210 bekannt, Sonnenlicht mit Hilfe eines Parabolspiegels auf eine lichtdurchlässige Rohrleitung zu richten, durch die eine Suspension von phototrophen Organismen geleitet wird. Der Parabolspiegel hat dabei zylindrische Gestalt und ist mit einer Nachführeinrichtung versehen. Bei dieser Einrichtung ist jedoch zu befürchten, daß mit der Konzentration des Sonnenlichts auch eine zu hohe Erwärmung der Suspension eintritt, was zu einer Schädigung der lebenden Organismen führen kann.

Weiterhin ist nach der GB-AS 2 191 195 ein Bioreaktor bekannt, bei dem neben einer Belichtungsmöglichkeit des Kulturmediums mittels Sonnenlicht auch eine solche mittels Kunstlicht vorgesehen ist. Das Licht wird hierbei über einen optischen Lichtleiter in den Reaktionsbehälter eingeleitet. Hier ist eine große Anzahl von Lichtleitern vorgesehen, die sich in transparenten Rohren befinden, die sämtlich ihre Lichtenergie über den einen in den Reaktor geführten optischen Lichtleiter erhalten.
Infolge der dichten Anordnung der transparenten Rohre im Reaktionsbehälter können nur solche Algen kultiviert werden, die keine Exo-Polysaccharide ausscheiden. Diese würden sich an den transparenten Rohren absetzen und die Lichtleistung des Reaktors nach und nach so stark vermindern, daß eine wirtschaftliche Kultivierung von Algen gänzlich unmöglich würde. Außerdem gestaltet sich die Reinigung des Reaktionsbehälters äußerst schwierig. Eine saubere und keimfreie Analge ist jedoch die Voraussetzung für das Gelingen der Kultivierung.

Nach der US-PS 3958364 ist eine Einrichtung zur Kultivierung von Algen bekannt, bei der ein großes, quaderförmiges Gefäß von wenigstens drei seiner Längsseiten her belichtet wird. Dabei wird in das Gefäß CO₂ und Luft vom Boden her eingeleitet.

Da keine anderweitige Möglichkeit zur Bewegung des Kulturmediums besteht, wird hierdurch eine gewisse Bewegung desselben hervorgerufen, die jedoch so gering ist, daß man im wesentlichen von einem stillstehenden Kulturmedium sprechen kann. Hierdurch wird jedoch eine sehr ungleichmäßige Belichtung desselben erreicht. Am intensivsten werden demnach die Randzonen des Gefäßes belichtet, während nach der Mitte zu ein ganz erheblicher Abfall der Lichtintensität eintritt, was dazu führt, daß das Wachstum der Algen nur sehr langsam erfolgt oder ganz stagniert.
Nach der DD-PS 152 807 ist weiterhin eine Vorrichtung für die Kultivierung autotropher Mikroorganismen bekannt, die sowohl mit Sonnenlicht als auch mit Kunstlicht betriebbar ist. Hierbei ist ein als Spirale ausgebildeter Exponator vorgesehen, der in der Horizontalen liegt, der sowohl vom Sonnenlicht bestrahlt werden kann als auch vom Kunstlicht. Da der Exponator stets in der Horizontalen verbleibt, verändert sich der Einfallswinkel des Sonnenlichtes ständig, wodurch sich auch die Strahlungsintensität ständig verändert. Besonders bei kleinem Einfallswinkel ist die Intensität der Strahlen gering. Da der Exponator eine verhältnismäßig große Fläche bedeckt, macht sich eine hohe Leuchtdichte im Falle der Verwendung von Kunstlicht erforderlich, wodurch der Kultivationsprozeß und damit das gewonnene Endprodukt erheblich verteuert wird.

Die GB-AS 2 118 572 hat ebenfalls eine Einrichtung zur Kultivierung von phototrophen Mikroorganismen zum Inhalt, bei der ein mäanderförmig gebogenes Rohr als Exponator vorgesehen ist. Dabei wird dasselbe entweder mittels Sonnenlicht bestrahlt oder es erfolgt eine Belichtung mittels Quecksilberdampflampfen. Auch hier erfolgt eine nur mäßige Ausnutzung der eingesetzten Lichtenergie, wodurch sich das Herstellungsverfahren einerseits zeitaufwendig und andererseits kostenintensiv gestaltet.

Der Erfindung liegt eine Aufgabe zugrunde, eine Einrichtung zum Kultivieren von autotrophen Mikroorganismen zu schaffen, die es gestattet, mit der aufgewandten Lichtenergie eine möglichst große Fläche zu belichten, wobei die Einrichtung raumsparend aufgebaut sein soll.

Erfindungsgemäß wird die Aufgabe dadurch gelöst, daß wenigstens zwei ineinander angeordnete, aus transparentem Material bestehende, beliebig kombinierbar verknüpfbare Rohrwendeln vorgesehen sind, deren parallele Längsseiten über Krümmer miteinander verbunden sind, wobei sich zwischen den parallelen Längsseiten Lichtquellen befinden und das ganze in einem von seiner äußeren Begrenzung her quaderförmigen Rahmengestell gelagert ist. Hierbei sind die Rohre mit einer nach ihrer unteren Anschlußstelle gerichteten Neigung von etwa 1 % angeordnet.
Als Lichtquellen dienen Niederspannungs-Leuchtstoffröhren mit einem Wellenlängenbereich von 400 bis 750 nm. Dabei ist die Vielzahl der Lichtquellen als geschlossener Baustein ausgebildet, der austauschbar und/oder teilweise abschaltbar ausgebildet ist.
Dabei ist unter dem Ausdruck "ineinander angeordnete Rohrwendeln" zu verstehen, daß eine beliebige Anzahl von eine flache Form aufweisenden Rohrwendeln derart zueinander angeordnet sind, daß ihre Längsseiten mit geringstem Zwischenraum parallel zueinander verlaufen, in der gleichen Ebene liegen und nach außen wie innen eine Fläche bilden. Die Rohre sind dabei durch Krümmer verbunden, die entsprechend zu bemessen sind.
Durch die erfindungsgemäße Einrichtung wird erreicht, daß die eingesetzte Lichtenergie weitestgehend ausgenutzt wird, da die Lichtquellen gleichzeitig zwei aus Rohren gebildete Flächen belichten. Infolge der Ineinanderanordnung mehrerer Rohrwendeln, wodurch die einzelnen Rohre in sehr geringem Abstand zueinander verlaufen, wird es möglich, das Kultivierungsmedium über einen sehr langen Zeitraum an den Lichtquellen vorbeizuführen. Durch Hintereinanderschaltung von beispielsweise drei Rohrwendeln, die im Rahmengestell ineinander angeordnet sind, läßt sich die Durchlaufstrecke um das Mehrfache vergrößern. Hierzu werden dieselben durch entsprechende abnehmbare Krümmer in genannter Weise miteinander verbunden.
Die Anordnung der Rohrwendeln in einem Rahmengestell, wodurch sie ein leicht transportables, geschlossenes Ganzes bilden, läßt die erfindungsgemäße Einrichtung zu einem austauschbaren Baustein werden, wodurch es auch ermöglicht wird, beliebig große Anlagen zur Kultivierung von autotrophen Mikroorganismen durch Hintereinanderschaltung dieser Bausteine aufzubauen, die verhältnismäßig wenig Raum beanspruchen.
Durch die Neigung der Rohre nach ihrer unteren Anschlußstelle hin, ist es möglich, die Einrichtung nach der Reinigung völlig auslaufen zu lassen. Dadurch werden Rückstände von Reinigungsmitteln sowie -flüssigkeiten vermieden, die auf den weiteren Betrieb einen schädigenden Einfluß ausüben könnten.

Die Reinigung erfolgt mittels Schaumgummibällen, die entsprechend der lichten Weite des Rohres bemessen sind und mit Hilfe der Reinigungsflüssigkeit durch die Rohrwendeln gedrückt werden.
Ein besonderer Vorteil der erfindungsgemäßen Einrichtung ist es, eine Volumenvergrößerung oder -verkleinerung ohne weiteres dadurch zu ermöglichen, daß die ineinander angeordneten Rohrwendeln entweder hintereinandergeschaltet oder voneinander getrennt werden. Durch diese Maßnahme wird es möglich, in einer einzelnen Rohrwendel die Vorkultivierung bis zur Größenordnung des Startvolumens vorzunehmen. Dadurch werden alle Anlagen zur Vorkultivierung der autotrophen Mikroorganismen überflüssig und können wegfallen.

Die Lichtquellen sind blockartig zueinander angeordnet und so als ein geschlossener Baustein ausgebildet, der nach Bedarf austauschbar ist. Hierdurch wird es möglich, dieselben gegen solche einer anderen Wellenlänge des Lichtes auszutauschen, so daß die für die jeweiligen Kulturen günstigen Bedingungen geschaffen werden können. Dadurch werden auch Ausbeuten erzielt, die weit über den mit bisher bekannten Einrichtungen erzielbaren Ergebnissen liegen.
Die Erfindung ist anhand eines Ausführungsbeispieles näher erläutert.
In der zugehörigen Zeichnung zeigen:
- Fig. 1: eine Seitenansicht der erfindungsgemäßen Einrichtung
- Fig. 2: dieselbe in Draufsicht
- Fig. 3: eine Ansicht in Richtung A.

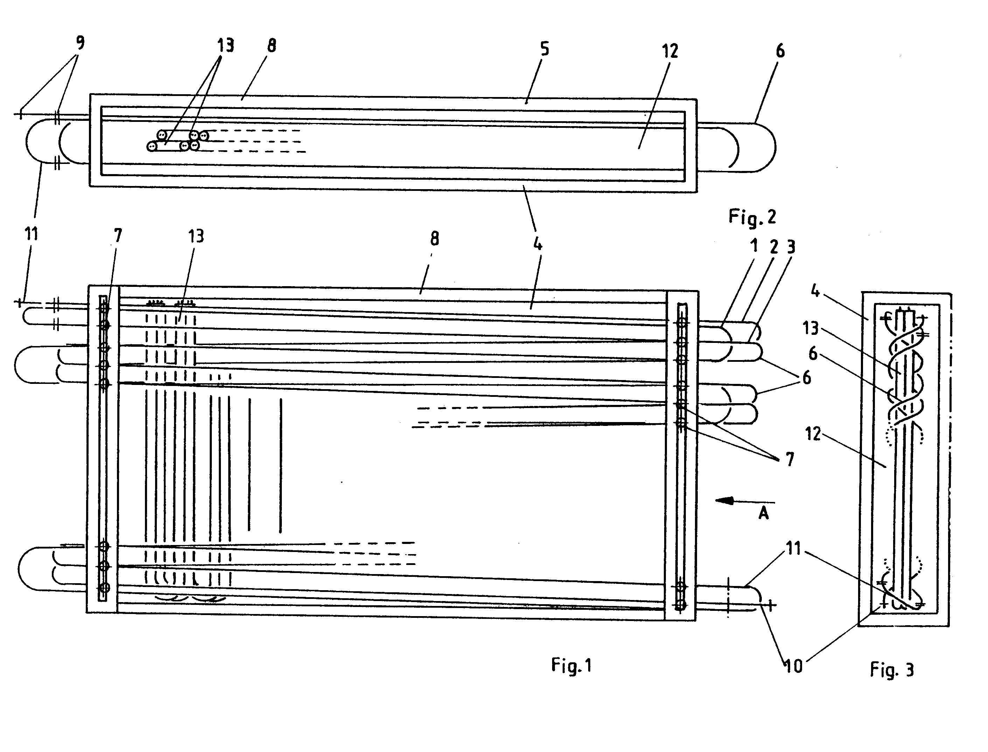

Wie in Fig. 1 dargestellt, sind die Rohrwendeln 1, 2 und 3 ineinander angeordnet. Dadurch liegen die Rohre an den beiden Längsseiten 4 und 5 sehr dicht beieinander. Dabei sind die Rohre über Krümmer 6 fest miteinander verbunden und so zu Wendeln ausgebildet.

Die Rohrwendeln 1, 2 und 3 sind an den Halterungen 7 fest im Rahmengestell 8 fixiert. Die einzelnen Rohre der Rohrwendeln 1, 2 und 3 sind mit einer geringen Neigung von etwa 1 % nach dem Anschlußstutzen 10 hin im Rahmengestell 8 angeordnet.
Durch die lösbaren Flanschverbindungen 9 und die dadurch abnehmbaren Krümmer 11 ist es möglich, die drei Rohrwendeln 1, 2 und 3 hintereinander zu schalten.
Werden die abnehmbaren Krümmer 11 entfernt, so bildet jede Rohrwendel 1, 2 und 3 ein durchgängiges System, das unabhängig von den jeweils beiden anderen ist. Demzufolge ist eine beliebig kombinierbare Verknüpfung der Rohrwendeln miteinander möglich.
In dem von den Längsseiten 4 und 5 gebildeten Hohlraum 12, dessen Breite durch die Krümmer 6 bestimmt wird, befinden sich die Lichtquellen, die als Leuchtstoffröhren 13 ausgebildet sind.
Zweckmäßigerweise sind die Leuchtstoffröhren 13 U-förmig gebogen, so daß nur am oberen Ende eine Anschlußmöglichkeit vorhanden sein muß. Hierdurch wird es ermöglicht, daß die Lichtquellen 13 als gesonderter Baustein ausgebildet werden können und so leicht entfernbar und auswechselbar sind.

Zum Betrieb der Einrichtung wird dieselbe am Anschlußstutzen 10 mit einer entsprechenden Zuleitung verbunden, durch welche eine Suspension zugeführt wird, in welche die entsprechenden Mikroorganismen inokuliert worden sind. Dazu wird Luft und CO₂ zugeführt. Bis zur Erreichung des Startvolumens wird die Suspension zunächst nur über eine der Rohrwendeln 1, 2 oder 3 umgepumpt. Ist nun in dieser zunächst nur mit einer Rohrwendel betriebenen Einrichtung das Startvolumen erreicht, können zunächst eine weitere Rohrwendel und anschließend auch die dritte Rohrwendel angeschaltet werden.

Die in Rohrwendel vorwärts strömende Suspension wird in Rohrwendel 2 gegenläufig hierzu geführt und gelangt dann nach Rohrwendel 3, wo es wieder in der Richtung wie in Rohrwendel 1 strömt und am entsprechenden Anschlußstutzen 10 wieder die Einrichtung verläßt und anschließend über eine nicht dargestellte Pumpe erneut in die Rohrwendeln 1, 2 und 3 gedrückt wird. Die erforderliche Lichtenergie wird über die Leuchtstoffröhren 13 zugeführt, die sehr dicht an den Rohren angeordnet sind. Infolge ihrer Anordnung in dem Hohlraum 12 wird die Lichtenergie nach zwei Seiten hin wirksam. Hierdurch wird die Lichtenergie besonders gut ausgenutzt. Die dichte Anordnung der Leuchtstoffröhren 13 an den Rohrwendeln 1, 2 und 3 erbringt den höchstmöglichen Wirkungsgrad des Lichtes. Dabei wirkt es sich günstig aus, daß die von den Leuchtstoffröhren 13 erzeugte Temperatur vernachlässigbar gering ist. Die Suspension durchströmt außerdem ständig einen Wärmeaustauscher, wodurch unzulässige Erwärmungen der strömenden Suspension vermieden werden. Wird die volle Lichtintensität nicht benötigt, ist es möglich, einige Leuchtstoffröhren 13, beispielsweise jede zweite oder jede dritte, abzuschalten. Auf diese Weise ist die Vorrichtung stets mit hoher Effizienz betreibbar.

## Patentansprüche

1. Einrichtung zum Kultivieren von autotrophen Mikroorganismen, dadurch gekennzeichnet, daß wenigstens zwei ineinander angeordnete, aus transparentem Material bestehende, beliebig kombinierbar verknüpfbare Rohrwendeln (1, 2, 3) vorgesehen sind, deren parallele Längsseiten (4, 5) über Krümmer (6, 11) miteinander verbunden sind, wobei zwischen den parallelen Längsseiten (4, 5) Lichtquellen (13) sich befinden, und das Ganze in einem von seiner äußeren Begrenzung her quaderförmigen Rahmengestell (8) gelagert ist.

2. Einrichtung nach Anspruch 1, dadurch gekennzeichnet, daß die Rohre mit einer nach ihrer unteren Anschlußstelle (10) gerichteten Neigung angeordnet sind.

3. Einrichtung nach Anspruch 1 und 2, dadurch gekennzeichnet, daß als Lichtquellen (13) Niederspannungs-Leuchtstoffröhren mit einem Wellenlängenbereich von 400 bis 750 nm vorgesehen sind.

4. Einrichtung nach Anspruch 1 bis 3, dadurch gekennzeichnet, daß die Vielzahl der Lichtquellen (13) als geschlossener Baustein ausgebildet sind, der austauschbar und/oder teilweise abschaltbar ausgebildet ist.

## Claims

1. Plant for the cultivation of autotrophic microorganisms, wherein at least two tubular coils (1, 2, 3), arranged into each other, of transparent material, in random configurations, are connected longitudinally (4, 5) by a knee (6, 11), light sources (13) are arranged between the parallel longitudinal sides (4, 5), and the whole unit is carried by a square framework (8).

2. Plant according to claim 1, wherein the tubes are arranged at an inclination to the lower connecting branch (10).

3. Plant according to claims 1 and 2, wherein low-voltage fluorescent lamps of a wavelength range of 400 to 750 nm are used as light sources (13).

4. Plant according to claims 1 to 3, wherein the total number of light sources (13) is forming a modular unit which may be interchanged and/or partly switched off.

## Revendications

1. Installation pour la culture de microorganismes autotrophes, caractérisée en ce qu'il est prévu au moins deux serpentins (1, 2, 3) reliables et pouvant être combinés sous une forme quelconque, encastrés l'un dans l'autre, en un matériau transparent (1, 2, 3), et dont les côtés longitudinaux parallèles (4, 5) sont reliés entre eux par des coudes (6, 11), des sources lumineuses (13) se situant entre les côtés longitudinaux parallèles (4, 5) et l'ensemble étant monté dans un châssis (8) parallélépipède à partir de sa délimitation extérieure.

2. Installation selon la revendication 1, caractérisée en ce que les tubes sont agencés avec une déclivité dirigée vers leur point de raccordement inférieur (10).

3. Installation selon les revendications 1 et 2, caractérisée en ce qu'il est prévu en tant que sources lumineuses (13) des tubes luminescents basse tension avec une plage de longueur d'ondes de 400 à 750 nm.

4. Installation selon les revendications 1 à 3, caractérisée en ce que la pluralité de sources lumineuses (13) est conçue sous forme de module fermé qui est de conception interchangeable et/ou partiellement déconnectable.
